# EUROPEAN PATENT SPECIFICATION

(11) **EP 4 081 506 B1**
(45) Date of publication and mention of the grant of the patent: **14.02.2024**
(21) Application number: 20820950.2
(22) Date of filing: 11.12.2020
(51) Int. Cl.: C07C 407/00, C07C 409/20

(54) **PROCESS FOR THE PREPARATION OF HYDROPEROXY ALCOHOLS USING A HETEROGENOUS CATALYST**
VERFAHREN ZUR HERSTELLUNG VON HYDROPEROXYALKOHOLEN UNTER VERWENDUNG EINES HETEROGENEN KATALYSATORS
PROCÉDÉ DE PRÉPARATION D'HYDROPEROXYALCOOLS À L'AIDE D'UN CATALYSEUR HÉTÉROGÈNE

(30) Priority: 23.12.2019 EP 19306758
(43) Date of publication of application: 02.11.2022
(73) Proprietor: Demeta, 35000 Rennes (FR); Université de Rennes, 35042 Rennes (FR)
(72) Inventor: COTA, Iuliana Maria, 41009 SEVILLA (ES); CAIJO, Frédéric, 35235 THORIGNE-FOUILLARD (FR); DARCEL, Christophe, 35000 RENNES (FR); ESCANDE, Vincent, 35000 RENNES (FR)
(74) Representative: Renard, Emmanuelle
(86) International application number: PCT/EP2020/085669
(87) International publication number: WO 2021/130028

(56) References cited:
- EP-A1- 1 618 953
- LI, P-H.; LI, B-L.; AN,J Z-M.; MO, L-P.; CUI, Z-S.; ZHANG, Z-H.: "Magnetic Nanoparticles (CoFe2O4)-Supported Phosphomolybdate as an Efficient, Green, Recyclable Catalyst for Synthesis of beta-Hydroxy Hydroperoxides", ADVANCED SYNTHESIS AND CATALYSIS, vol. 355, 9 October 2013 (2013-10-09), pages 2952-2959, XP002802107, cited in the application

## Description

### FIELD OF THE INVENTION

The present invention relates to a process for preparing hydroperoxy alcohols using aqueous hydrogen peroxide as an oxidant in a solvent selected from water-soluble carboxylic acids, in the presence of a metallic mixed oxide heterogeneous catalyst. It also pertains to the use of this catalyst in the synthesis of hydroperoxy alcohols.

### BACKGROUND OF THE INVENTION

Hydroperoxy alcohols are useful precursors for the synthesis of aldehydes and carboxylic acids. Moreover, they are also important intermediates for the synthesis of 1,2,4-trioxanes which are known as antimalarial agents [C. Singh, H. Malik, S.K. Puri, "Orally Active 1,2,4-Trioxanes: Synthesis and Antimalarial Assessment of a New Series of 9-Functionalized 3-(1-Arylvinyl)-1,2,5-trioxaspiro[5.5]undecanes against Multi-Drug-Resistant Plasmodium yoelii nigeriensis in Micel" J. Med. Chem. 2006, 49, 2794-2803]. Several processes for the synthesis of the hydroperoxy alcohols have been described until now in the literature.

In the study of Payne and Smith hydroxylation of cyclohexene in tert-butyl alcohol employing H₂WO₄ as catalyst leading to formation of organic peroxide and diol is described [G. B. Payne, C. W. Smith, "Tungstic Acid catalyzed Hydroxylation of Cyclohexene in Nonaqueous Media" J. Org. Chem. 1957, 22, 1682-1685]. After 2 h of reaction at 60 °C with 4 wt % catalytic loading, only 65 % of cyclohexene was converted into 20 % organic hydroperoxide and 50 % diol. Although the catalytic loading was high (4 wt %) an incomplete conversion (65%) and low hydroperoxy alcohol yield (20%) were obtained.

Production of hydroperoxy alcohols by X-radiation of thymine in aqueous aerated solutions was reported by Ekert and Monier [B. Ekert, R. Monier, "Structure of Thymine Hydroperoxide produced by X-lrradiation" Nature 1959, 5, 184, B.A.58-B.A.59]. Though the thymine conversion was complete, only 10 % yield of hydroperoxy alcohol was obtained. Moreover, at industrial scale X-radiation cannot be applied.

Mattuci et al. described the oxidation of isobutene with anhydrous H₂O₂ catalyzed by molybdenyl acetylacetonate (C₁₀H₁₆MoO₆; 2.5 mol; 0.48 wt %) at 45 °C. In these conditions only around 50 % conversion of isobutene was obtained. Moreover, the maximum yield of 2-hydroperoxy-2-methylpropan-1-ol obtained after 6 h was 20 % [A. M. Mattucci, E. Perrotti, A. Santambrogio, "Epoxidation Reaction with Anhydrous Hydrogen Peroxide" Chem. Commun., 1970, 1198-1199].

Oxidative cleavage of 14 olefins with H₂O₂ catalyzed by methyltrioctylammonium tetrakis(oxodiperoxotungsto)phosphate (1 mol% catalytic loading) under two-phase conditions was reported in the study of Antonelli et al. [E. Antonelli, R. D'Aloisio, M. Gambaro, T. Fiorani, C. Venturello, "Efficient Oxidative Cleavage of Olefins to Carboxylic Acids with Hydrogen Peroxide Catalyzed by Methyltrioctylammonium Tetrakis(oxodiperoxotungsto)phosphate(3-) under Two-Phase Conditions. Synthetic Aspects and Investigation of the Reaction Course" J. Org. Chem. 1998, 63, 7190-7206]. After 30 min of reaction at 85 °C, good results were obtained in terms of olefin conversion (90-99 %) depending on the nature of the olefin; the yields of the β-hydroperoxy alcohols obtained as reaction intermediates ranged between 22-63 %.

Patent JP2003342255 describes the oxidative cleavage of oleic acid methyl ether into 9(10)-hydroperoxy-10(9)-hydroxy stearic acid methyl ester with H₂O₂ (40 w/v, 2 equiv.) employing different catalysts (H₂WO₄, MoOs, H₃PMo₁₂O₄₀, MoO₃·H₂O, H₂WO₄) with 10 mol % catalytic loading. After 5 h of reaction at 35 °C, the oleic acid methyl ether conversion ranged between 11-75 % and the 9(10)-hydroperoxy-10(9)-hydroxy stearic acid methyl ester yield between 4-50 % depending on the nature of the catalyst. When the temperature was increased to 70 °C after 1 h of reaction using H₂WO₄ as the catalyst, a complete conversion and 37 % yield of 9(10)-hydroperoxy-10(9)-hydroxy stearic acid methyl ester were obtained.

However, all these processes present one or several drawbacks such as: (i) low substrate conversion and product yield are obtained; (ii) the catalysts are homogeneous and therefore not reusable after the first use; (iii) the use of methods which cannot be applied at industrial scale (X-radiation); (iv) the use of high reaction temperature (60-85 °C) and thus energy input. Regarding the industrial production of hydroperoxy alcohols, it is important for environmental and economic considerations to be able to perform this reaction in milder conditions, with a recyclable catalyst.

Only one publication reports the use of a heterogeneous catalyst for the production of hydroperoxy alcohol. In the study of Li et al. [P.-H. Li, B.-L. Li, Z.-M. An, L.-P. Mo, Z.-S. Cui, Z.-H. Zhang, "Magnetic Nanoparticles (CoFe2O4)-Supported Phosphomolybdate as an Efficient, Green, Recyclable Catalyst for Synthesis of β-Hydroxy Hydroperoxides", Adv. Synth. Catal. 2013, 355, 2952 - 2959], magnetic nanoparticles-supported phosphomolybdate was used as recyclable heterogeneous catalyst for the synthesis of hydroperoxy alcohols with up to 8 consecutive runs with notably a slow decreasing of activity. However, this catalyst worked only with ethereal hydrogen peroxide, an extremely hazardous oxidant, likely to cause explosions, and whose use in laboratory is limited to small quantities, behind safety shields [Y. Li, H.-D. Hao, Q. Zhang, Y. Wu, "A Broadly Applicable Mild Method for the Synthesis of gem-Diperoxides from Corresponding Ketones or 1,3-Dioxolanes", Org. Lett. 2009, 11, 1615-1618]. Because of its hazardous properties, ethereal hydrogen peroxide cannot be used in industrial applications for safety reasons. It is thus still necessary to develop a new system involving a heterogeneous catalyst, able to perform the synthesis of hydroperoxy alcohols in mild conditions, with industry-compatible oxidants, like aqueous hydrogen peroxide.

The advantages of the proposed technology are: (i) complete conversion and acceptable product yield; (ii) the use of a heterogeneous catalyst which can be recycled and reused many times without significant loss of activity; (iii) possibly low reaction temperature; (iv) the use of aqueous hydrogen peroxide in industry-compliant conditions.

### SUMMARY OF THE INVENTION

This invention is directed to a process for preparing hydroperoxy alcohols (also named "beta-hydroxy hydroperoxides") with the following formula (Ia) and/or (Ib):

R¹-CR⁴(OOH)-CR³(OH)-R² (Ia)

R²-CR³(OOH)-CR4(OH)-R¹ (Ib)

wherein R¹ and R² are each independently an optionally substituted alkyl group with 1 to 20 carbon atoms or a -L-A group wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a-(CH₂)-CH(OCOR⁵)-CHOCOR⁶ group, with R⁵ and R⁶ being independently a C8-C22 linear or branched alkyl group which is optionally substituted by at least one hydroperoxy (-OOH) or hydroxyl (-OH) substituent,
or R¹ and R² form together a carbocycle consisting of a 6 to 12 membered ring which is optionally substituted.
R³ and R⁴ are each independently a hydrogen atom, an optionally substituted alkyl group with 1 to 20 carbon atoms or a group -L-A wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a -(CH₂)-CH(OCOR⁵)-CHOCOR⁶ group, with R⁵ and R⁶ being independently a C8-C22 linear or branched alkyl group which is optionally substituted by at least one hydroperoxy or hydroxyl substituent,
comprising reacting at least one functionalized or unfunctionalized, linear or cyclic, olefin which comprises at least one carbon - carbon double bond with aqueous hydrogen peroxide in a solvent selected from water-soluble carboxylic acids, in the presence of a heterogenous catalyst which is a metallic mixed oxide having formula (II):

   MₓM'_{y}O_{z} (II)
wherein:
   M is a metal;
   M' is W or Mo,
   x is a number from 1 to 10, preferably from 1 to 5, more preferably from 1 to 2;
   y is a number from 1 to 10, preferably from 1 to 5, more preferably from 1 to 3;
   z is a number from 1 to 40, preferably from 2 to 20, more preferably from 3 to 15.

This invention also pertains to the use of the above catalyst in the synthesis of hydroperoxy alcohols.

### DETAILED DESCRIPTION

In the process of this invention, at least one olefin is reacted with aqueous hydrogen peroxide in a specific solvent, in the presence of a metallic mixed oxide heterogenous catalyst.

The olefins used as a substrate may be functionalized or not, and may be linear or cyclic. These olefins typically have formula (III):

(R¹)(R⁴)C=C(R²)(R³) (III)

wherein R¹ and R² are each independently an optionally substituted alkyl group with 1 to 20 carbon atoms or a -L-A group wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a-(CH₂)-CH(OCOR⁵)-CHOCOR⁶ group, with R⁵ and R⁶ being independently a C8-C22 linear or branched alkyl group,
or R¹ and R² form together a carbocycle consisting of a 6 to 12 membered ring which is optionally substituted.
R³ and R⁴ are each independently a hydrogen atom, an optionally substituted alkyl group with 1 to 20 carbon atoms or a group -L-A wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a -(CH₂)-CH(OCOR⁵)-CHOCOR⁶ group, with R⁵ and R⁶ being independently a C8-C22 linear or branched alkyl group.

In a preferred embodiment, the functionalized olefin bears at least one carboxyl or alcoxycarbonyl group. The functionalized olefin can be chosen among mono- or polyunsaturated aliphatic carboxylic acids and their esters. Such carboxylic acids can comprise from 6 to 60 carbon atoms, preferably from 6 to 32 carbon atoms, more preferably from 12 to 24 carbon atoms, more preferably from 12 to 18 carbon atoms, and can have from 1 to 6 C=C unsaturations, more preferably from 1 to 3 C=C unsaturations.

Representative examples of aliphatic mono- or polyunsaturated carboxylic acids are lauroleic acid, myristoleic acid, palmitoleic acid, sapienic acid, petroselaidic acid, oleic acid, elaidic acid, petroselinic acid, vaccenic acid, gadoleic acid, cetoleic acid, erucic acid, selacholeic or nervonic acid, linoleic acid, linolenic acid, rumenic acid, stearidonic acid, eleostearic acid, catalpic acid, arachidonic acid, including their mixtures. The carboxylic acid can optionally be mono or poly-hydroxylated and notably can be ricinoleic acid. This acid can be obtained by a chemical or enzymatic hydrolysis of at least a fatty acid triglyceride from vegetable oil. As an alternative, it can be obtained from animal fat. On the other hand, the ester of the acid can be one or more triglycerides derived from a vegetable oil or it can be obtained by esterification of the corresponding acid or by transesterification of the corresponding triglyceride by a mono-alcohol. Representative examples of esters derived from aliphatic mono- or poly-unsaturated carboxylic acids are linear or branched C1-C6 alkyl esters, such as methyl, ethyl, n-propyl, iso-propyl, n-butyl, tert-butyl, pentyl, iso-pentyl or hexyl, without this list being exhaustive. One representative example of triglyceride is triolein. Preferentially, the functionalized olefin is selected from oleic acid, palmitoleic acid, erucic acid, linoleic acid, linolenic acid their esters and mixtures thereof. More preferably, the functionalized olefin is oleic acid or one of its esters, more particularly methyl oleate.

Representative examples of vegetable oils are wheat germ oil, sunflower oil, argan oil, hibiscus oil, coriander oil, grape seed oil, sesame oil, corned germ oil, apricot oil, castor oil, Shea oil, avocado oil, olive oil, peanut oil, soybean oil, sweet almond oil, palm oil, rapeseed oil, cotton oil, hazelnut oil, macadamia oil, jojoba oil, alfalfa oil, poppy oil, squash oil, blackcurrant oil, lavender oil, primrose oil, borage oil, millet oil, barley oil, quinoa oil, rye oil, safflower oil, candlenut oil, passionflower oil, rose hip oil, Echium oil, camelina oil or camellia oil. Alternatively, one or several oils from microalgae biomass can be used.

In a particular embodiment of the process, at least one of R¹ and R² (preferably, both R¹ and R²) are different from a hydrogen, and R³ and R⁴ are as defined herein. In such an embodiment, it is preferred that R³ and R⁴ are hydrogen.

In another particular embodiment of the process, R¹ is a -L-A group with L being a bond and A being a hydrogen, R⁴ is a hydrogen, and R² and R³ are as defined herein.

In such an embodiment, it is preferred that one of R² and R³ is different from a hydrogen, and the other one of R² and R³ is a hydrogen.

In another embodiment of the process, cyclic olefins can be used as starting materials. Representative examples of cyclic olefins which can be used in this invention are cyclohexene, cycloheptene, cyclooctene, cyclononene, cyclodecene, cycloundecene, cyclododecene, dicyclopentadiene, norbornene and norbornadiene, without this list being exhaustive.

In the following description, the term « substrate » will be used in order to designate functionalized or unfunctionalized, cyclic or linear olefins which will be used as starting material in the process of this invention.

Irrespective of the substrate being used, the present process involves the transformation of the olefin substrate into a hydroperoxy alcohol (HPA) in the presence of hydrogen peroxide as an aqueous solution in a solvent selected from water-soluble carboxylic acids and a catalytic loading of a heterogeneous metallic mixed oxide catalyst.

The concentration of the solution of hydrogen peroxide used is at least 30%, or at least 45% and at most 60% or even 70%, for example 30%, 45%, 60% or 70% (m/v). Preferably, said concentration is at least 45 % and at most 70 % (m/V). The quantity of hydrogen peroxide (for instance, having a concentration of 60% (m/V)) used in the process ranges from 2 to 10 equivalents, preferably from 3.5 to 4 equivalents and more preferably is about 3.5 equivalents.

The catalyst employed in this process is a heterogenous catalyst, i.e. is present in the reaction medium in the form of particles having a size preferably comprised between 1 nm and 1000 µm and more preferably from 10 nm to 10 µm, as measured by Transmission Electron Microscopy (TEM). This catalyst comprises a mixture of metal oxides and can be described by formula (II):

MₓM'_{y}O_{z} (II)

wherein:
M is a metal;
M' is W or Mo,
x is a number from 1 to 10, preferably from 1 to 5, more preferably from 1 to 2;
y is a number from 1 to 10, preferably from 1 to 5, more preferably from 1 to 3;
z is a number from 1 to 40, preferably from 2 to 20, more preferably from 3 to 15.

The catalyst may be prepared according to well-known procedures and typically by mixing two aqueous solutions, one of which contains a salt of M and the other of which contains a salt of M'. Any organic or inorganic salt may be used for this purpose, such as nitrate, halide, carboxylate, sulfate, or perchlorate salts of M and halide of M' or oxyhalide of M'. Alternatively, the salt of M' may be a tungstate or molybdate salt, for instance with sodium, potassium or ammonium.

Following its preparation process, the catalyst is usually in the form of an aqueous suspension or slurry.

It may be used in this invention under its native form, its calcined form or its hydrothermal form. The native form is obtained by filtering the catalyst suspension in order to recover the catalyst which is then optionally washed with water and further dried at 80-150 °C, preferably at 100-150 °C, more preferably at about 120 °C for 12 h under air.

The calcination form is obtained by filtering the catalyst suspension, in order to recover the catalyst which is then optionally washed with water and further subjected to heat treatment at 160 - 1200 °C, preferentially at 300-700 °C, more preferentially at about 500 °C, under air, with heating rates preferably between 1 and 100 °C/min, preferably from 5 to 25 °C/min., more preferably at about 10 °C/min.

The hydrothermal form is obtained by hydrothermal treatment of the catalyst suspension in an autoclave at 50-300 °C, preferably at 100-250 °C, more preferably at 150-200 °C, usually for 1-48 h, preferably for 5-24 h, more preferably for 10-14 h. The catalyst suspension obtained may then be subjected to several centrifugation-washing steps with water before being dried at 80-150 °C, preferably at 100-150 °C, more preferably at about 120 °C for 12 h under air.

In this invention, use is preferably made of the catalyst in its hydrothermal form.

According to a preferred embodiment of this invention, the metal M is such that its cation forms a Lewis acid (as described for instance in Yamamoto, Hisashi. Lewis Acids in Organic Synthesis. Weinheim: Wiley-VCH, 2002). The metal M is preferably selected from the group consisting of alkaline earth metals, transition metals, post-transition metals, lanthanides and rare earth elements. More preferably, M is selected from Zn, Yb, Y, Bi, Al, Zr, still more preferably it is selected from Zn, Yb, Y, Bi, and still better M is Zn.

Examples of catalysts of formula (II) include, but are not limited to, ZnWO₄, Yb₂(WO₄)₃, Y₂(WO₄)₃, Al₂(WO₄)₃, Bi₂(WO₄)₃, Zr(WO₄)₂, MnMoO₄, ZnMoO₄, Yb₂(MoO₄)₃, Al₂(MoO₄)₃, Bi₂(MoO₄)₃, Ce₂(MoO₄)₃, Y₂(MoO₄)₃, or Zr(MoO₄)₂. Preferred catalysts of formula (II) are ZnWO₄, Yb₂(WO₄)₃, Y₂(WO₄)₃, Bi₂(WO₄)₃, or ZnMoO₄. A more preferred catalyst of formula (II) is ZnWO₄.

The HPA formation can be conducted using 0.1-20 wt%, preferably 1-20 wt%, more preferably 5-10 wt%, even more preferably 7-9 wt% of the mixed oxide catalyst (whatever its form, native, calcined or hydrothermal), relative to the weight of the olefin. In an embodiment where the mixed oxide catalyst is ZnWO₄, the HPA formation can be conducted using 0.1-20 mol%, preferably 5-10 mol%, relative to the weight of the olefin.

The cited reagents (olefin, aqueous hydrogen peroxide) and catalyst can be introduced in the reaction vessel in any order. The oxidative process is performed in a solvent selected from water-soluble carboxylic acids such as acetic acid and formic acid, preferably acetic acid. The quantity of solvent used typically ranges from 0.1 to 100 mL/mmol of olefin, preferably from 0.5 to 10 mL/mmol of olefin and more preferably from 0.9 to 1 mL/mmol of olefin. The reaction medium may comprise at least one additional organic solvent, for instance acetonitrile or an alcohol such as ethanol or t-butanol. The volume ratio of the additional organic solvent to the water-soluble carboxylic acid may in particular be from 1:100 to 10:1, preferably from 1:10 to 1:1.

Preferably, the reaction medium does not contain any other constituent than the olefin(s), hydrogen peroxide, water, the water-soluble carboxylic acid(s), the catalyst, and optionally the at least one additional organic solvent.

The process can be performed at a temperature of from 0 to 120 °C, preferably of from 0 to 90 °C, more preferably of from 20 to 60 °C and even more preferably of from 25 to 35 °C.

In case the catalyst is used in its native form, the reaction time usually ranges from 1 h to 48 h, preferably from 4 h to 24 h.

The catalyst may be recovered once the reaction is complete, and then recycled. The process of this invention thus preferably comprises the steps of:
- separating the catalyst from the reaction medium, washing it with an alcohol, wherein the centrifugation and washing steps may be repeated 1-10 times, and optionally drying the catalyst, and
- recycling the catalyst in a process as defined in claim 1.

For this purpose, the catalyst may be separated from the reaction medium by any means, such as by centrifugation (at 1000-15000 rpm, and more preferably at 6000 rpm during 1 min-1 h, and more preferably 20 min), washed with an alcohol such as ethanol. The centrifugation and washing steps may be repeated 1-10 times, and more preferably 3 times. The catalyst may then be dried if needed, as described above, before reusing in the next reaction run without notable loss of activity and chemoselectivity.

In the above washing step, any organic solvent (for instance, acetone, acetonitrile, ethyl acetate, or tetrahydrofuran) may be used instead of an alcohol.

This process of this invention can be used for the oxidative cleavage of unsaturated vegetable oils, more particularly to form fatty aldehydes and/or fatty acids. The transformation of the HPA obtained following the present process in aldehydes and /or carboxylic acids can be done by already described procedures via acidic or basic or radical catalytic pathways, or thermal pathways.

### EXAMPLES

The following working examples are provided for illustration purposes only and are not intended to restrict in any way the scope of this invention, which is defined by the attached claims.

### Materials and methods

The reagents were purchased from Sigma-Aldrich-Merck, Acros, Alfa-Aesar, Fisher, TCI, Fluorochem and were used without further purification. All reactions were carried out under air at atmospheric pressure. All reactions were performed in duplicate.

NMR: ¹H-NMR was used to monitor the reactions and to determine the structure of the formed products. The spectra were collected at room temperature (25 °C) using an AVANCE 400 NMR spectrometer at 400.1 MHz (Bruker). Upon completion of the reaction, 1,4-dibromobenzene was added as internal standard and the sample was analyzed with quantitative ¹H-NMR method (qNMR). All samples were prepared in chloroform-d (CDCl₃) by mixing 5 µL of sample with 500 µL of CDCl₃. The chemical shifts were expressed in ppm (parts per million) relative to the signal of residual CDCl₃. Data were processed using MestReNova software (Mastrelab Research S.L).

IR-FT: Solid-state IR-FT analysis were recorded between 400 and 4000 cm⁻¹ in an ATR mode using a Shimadzu IR Affinity-1 spectrometer.

N₂ adsorption / desorption: Nitrogen adsorption measurements were carried out with a Micromeritics Gemini VI instrument at 77 K. Prior to adsorption, the materials were out-gassed under vacuum at 250 °C for 2 h. Their surface area (S_{BET}) was calculated according to the Brunauer- Emmett-Teller (BET) method. The total pore volume (V) was determined from the amount of vapor adsorbed at p / p° = 0.99 and average pore diameter was determined assuming a cylindrical shape using 4V/S_{BET} formula.

Transmission electron microscopy (TEM): Transmission Electron Microscopy was performed on a JEOL 2100 LaB6 instrument operating at 200 kV and equipped with Orius SC 200D camera and EDS Oxford 80 mm² SDD spectrometer. Preparation of the powder samples for TEM consisted on the grinding of the powder in absolute anhydrous ethanol followed by deposition for drying on a carbon coated copper grid.

Powder XRD: The crystal structure and purity of the samples were checked by powder XRD using a Bruker D8 Advance diffractometer working in the modified Bragg-Brentano geometry with a monochromatized Cu Kα₁ radiation (λ = 1.54059 Å) and equipped with a LynxEye fast detector. The patterns were measured in the angular range 5° ≤ 20 ≤ 80° every 0.02° with a collection time of 483 s/step.

### Example 1: Preparation of the ZnWO₄ catalyst

The ZnWO₄ catalyst was synthesized using a slightly modified protocol described in Appl. Catal. A Gen. 2014, 469, 165-172. In a typical procedure, Na₂WO₄-2H₂O (analytical grade 99 + %) (9.89 g, 30 mmol) and Zn(NO₃)₂·6H₂O (reagent grade 98%) (8.92 g, 30 mmol) were put in two beakers. Then, 75 mL of distilled water were added to each beaker and magnetically stirred to form a homogeneous solution (at room temperature ~20 °C). Afterwards, the solution of Na₂WO₄ was added dropwise to the Zn(NO₃)₂ solution, under magnetic stirring to form a white slurry. The slurry was kept under stirring at room temperature for 24 h and then the resulting precipitate was filtered and washed with distilled water. The precipitate was dried in air atmosphere at 120 °C during 12 h to obtain the catalyst ZnWO₄ as a white solid.

Using this protocol, the following mixed oxides were obtained: ZnWO₄, Yb₂(WO₄)₃, Y₂(WO₄)₃, Al₂(WO₄)₃, Bi₂(WO₄)₃, Zr(WO₄)₂, starting from Zn(NO₃)₂·6H₂O, Yb(NO₃)₃·5H₂O, YCl₃·6H₂O, AlCl₃·6H₂O, Bi(NO₃)₂·5H₂O, ZrCl₄, respectively.

### Example 2: Preparation of the ZnMoO₄ catalyst

The ZnMoO₄ catalyst was synthesized using a slightly modified protocol described in J. Catal. 2001, 202, 268-278. In a typical procedure, a solution of (NH₄)₆Mo₇O₂₄·4H₂O (analytical grade 99 +%) (0.4 M, 5.29 g in 75 mL of H₂O) was added dropwise to a solution of Zn(NO₃)₂·6H₂O (reagent grade 98%) (0.4 M, 8.92 g in 75 mL of H₂O) under stirring at 80 °C. The solution was maintained at pH 6.0 by adding NaOH solution (2 M, 20 g in 250 mL of H₂O). The slurry was kept under stirring at 80 °C for 3 h and then the resulting precipitate was filtered and washed with distilled water. The precipitate was dried in air atmosphere at 120 °C during 12 h to obtain the ZnMoO₄ catalyst as a white solid.

Using this protocol, the following mixed oxides were obtained: MnMoO₄, ZnMoO₄, Yb₂(MoO₄)₃, Al₂(MoO₄)₃, Bi₂(MoO₄)₃, Ce₂(MoO₄)₃, Y₂(MoO₄)₃, Zr(MoO₄)₂, starting from Mn(NO₃)₂·4H₂O Zn(NO₃)₂·6H₂O, Yb(NO₃)₃·5H₂O, AlCl₃·6H₂O, Bi(NO₃)₂·5H₂O, CeCl₃·7H₂O, YCl₃·6H₂O, ZrCl₄, respectively.

### Example 3: Preparation of the calcined-ZnWO₄ catalyst

The precursor obtained using a procedure analogous to that described in Example 1 was then calcined at 500 °C for 4 h (10 °C/min rate) to obtain the calcined catalyst c-ZnWO₄.

Using similar procedure, the following calcined mixed oxides c-ZnWO₄, c-Yb₂(WO₄)₃, c-Y₂(WO₄)₃, c-Al₂(WO₄)₃, c-Bi₂(WO₄)₃, c-Zr(WO₄)₂ were also prepared.

### Example 4: Preparation of the hydrothermal-ZnWO₄ catalyst

The catalyst h-ZnWO₄ was synthesized following a slightly modified protocol described in Adv. Funct. Mater. 2003, 13, 639-647. In a typical procedure, 2.5 mmol (820 mg) of Na₂WO₄-H₂O (analytical grade 99+ %) and 2.5 mmol (740 mg) Zn(NO₃)₂·6H₂O (reagent grade 98%) were put in two beakers. Then, 7 mL of distilled water was added to each beaker and both mixtures were magnetically stirred to form a homogeneous solution (at room temperature ~20 °C). Afterwards, the solution of Zn(NO₃)₂ was added dropwise to the Na₂WO₄ solution, under magnetic stirring and upon complete addition the suspension was stirred for approximately 5 min. The resulted suspension was transferred into a Teflon-lined stainless-steel autoclave. The autoclave was sealed and heated at 180 °C for 12 h. After cooling down at room temperature, the solid was centrifuged (6000 rpm for 20 min) and washed with distilled water. The centrifuging-washing step was repeated 3 times. Afterwards, the solid was in air atmosphere at 120 °C during 12 h to obtain the catalyst h-ZnWO₄ as a white solid (Yield = 94%).

This solid was characterized with several analytical techniques.

First, it was characterized by powder XRD. All Bragg peaks in the diffraction pattern of the h-ZnWO₄ sample were indexed with the crystal structure of monoclinic wolframite (space group P2/c, lattice parameters: a = 4.6826(2) Å, b = 5.7088(2) Å, c = 4.9230(2) Å and β = 90.541(2)°) and were in accordance with the literature data. [H. Kraus, V. B. Mikhailik, L. Vasylechko, D. Day, K. B. Hutton, J. Telfer, Yu. Prots "Effect of Ca doping on the structure and scintillation properties of ZnWO4" Phys. Stat. Sol. (a) 2007, 204, 730-736].

The solid was also characterized by IR-FT. Several peaks in the range of 450-1000 cm⁻¹ corresponding to the absorption bands existing in ZnWO₄ structure were identified. The peaks located at 474 cm⁻¹, 536 cm⁻¹, 647 cm⁻¹ and 704 cm⁻¹ were associated to bending and stretching bands in Zn-O and W-O, while the peaks located at 841 cm⁻¹ and 872 cm⁻¹ were attributed to bending and stretching vibration bands in Zn-W-O [M. Mancheva, R. Iordanova, Y. Dimitriev "Mechanochemical synthesis of nanocrystalline ZnWO4 at room temperature" J. Alloy. Compd. 2011, 509, 15-20].

Additionally, the solid was characterized by N₂ adsorption / desorption technique. The sample of h-ZnWO₄ showed Type IV adsorption isotherms (IUPAC classification) characteristic for mesoporous structure. The textural properties of the sample are presented in Table 1.

**Table 1: Textural properties of h-ZnWO₄**

| S_{BET} (m²/g) | Average pore diameter (nm) | V (cm³/g) |
|---|---|---|
| 30.17 | 14.68 | 0.11 |

| | | |
|---|---|---|
| S_{BET}: surface area; V: total pore volume | | |

The solid was finally characterized by TEM technique. The sample was formed of quasi-spherical nanoparticles with diameters between 40 and 60 nm.

Compositional analysis of h-ZnWO₄ obtained by EDS (Energy Dispersive X-ray Spectroscopy) demonstrated the presence of Zn and W. The atomic composition of the sample is given in Table 2.

**Table 2: Composition of b-ZnWO₄**

| Atomic % | Spectrum 1 | Spectrum 2 | Spectrum 3 |
|---|---|---|---|
| Zn | 41.06 | 39.28 | 41.69 |
| W | 58.94 | 60.72 | 58.31 |

### Example 5: Oleic acid oxidation to HPA (Hydroperoxy alcohol)

A 50 mL Teflon tube equipped with a magnetic stirrer was loaded with oleic acid (90 % purity) (250 mg, 0.8 mmol, 1.0 equiv.), H₂O₂ (60 % (m/V) (0.159 mL, 2.8 mmol, 3.5 equiv.), acetic acid (puriss ≥ 99.8 %) (0.75 mL) and 20 mg h-ZnWO₄ (0.63 mmol, 8 wt % with respect to the substrate). The mixture was heated at 30 °C under stirring (400 rpm) for 24 h. Upon completion of the reaction, 1,4-dibromobenzene (purity > 90%) (9.5 mg, 0.4 mmol) was added to the reaction mixture and then diluted with acetic acid (puriss ≥ 99.8 %) to 5 mL using a volumetric flask. The products yield was determined by qNMR.

The catalyst remaining at the end of this reaction was analyzed similarly to the procedures given in Example 4. The peaks in the XRD and IR-FT patterns were the same as those observed before reaction. In addition, the textural properties of the catalyst were similar to those of the catalyst before reaction, as appears from Table 3 below:

**Table 3: Textural properties of h-ZnWO₄ after the catalytic reaction**

| S_{BET} (m²/g) | Average pore diameter (nm) | V (cm³/g) |
|---|---|---|
| 23.08 | 22.60 | 0.13 |

| | | |
|---|---|---|
| S_{BET}: surface area; V: total pore volume | | |

Finally, the composition of the catalyst as obtained by EDS was as follows (Table 4).

**Table 4: Composition of h-ZnWO₄**

| Atomic % | Spectrum 1 | Spectrum 2 | Spectrum 3 | Spectrum 4 | Spectrum 5 |
|---|---|---|---|---|---|
| Zn | 45.60 | 43.46 | 43.34 | 40.37 | 43.05 |
| W | 54.40 | 56.54 | 56.66 | 59.63 | 56.95 |

### Example 6: Study of various catalysts

Using a procedure analogous to that described in Example 5, the following bimetallic mixed oxides (which were prepared as described in Example 1) were evaluated as catalysts: ZnWO₄, Yb₂(WO₄)₃, Y₂(WO₄)₃, Bi₂(WO₄)₃ and ZnMoO₄. The reaction was performed at 30 °C for 5 h (Table 5) and 24 h (Table 6) with oleic acid (250 mg, 0.8 mmol, 1.0 equiv.), in the presence of H₂O₂ (60 % (m/V), 0.159 mL, 2.8 mmol, 3.5 equiv.) in acetic acid (0.75 mL).

**Table 5: Results of bimetallic mixed oxides catalytic activity after 5 h at 30 °C.**

| Entry | Catalyst | OA conv. (%) | Epoxide (%) | 1,2-diol (%) | HPA **Ia/b** (%) |
|---|---|---|---|---|---|
| 1 | ZnWO₄ | >99 | 0 | 18 | 75 |
| 2 | Yb₂(WO₄)₃ | >99 | 0 | 29 | 68 |
| 3 | Y₂(WO₄)₃ | >99 | 17 | 20 | 44 |
| 4 | Bi₂(WO₄)₃ | 85 | 6 | 25 | 61 |
| 5 | ZnMoO₄ | 84 | 5 | 8 | 47 |

As shown in Table 5, the bimetallic mixed oxides allow to obtain a complete or almost complete conversion of oleic acid after 5 h at 30 °C. HPA was obtained in yields from 44 to 75 %, as a major product. The 1,2-diol of oleic acid was also obtained in yields from 8 to 29 %. The epoxide, mostly in trace amounts, was also observed with Y₂(WO₄)₃, Bi₂(WO₄)₃, and ZnMoO₄.

**Table 6: Results of bimetallic mixed oxides catalytic activity after 24 h at 30 °C.**

| Entry ID | Catalyst | OA conv. (%) | Epoxide (%) | 1,2- diol (%) | HPA **Ia/b** (%) |
|---|---|---|---|---|---|
| 1 | ZnWO₄ | >99 | 0 | 22 | 76 |
| 2 | Yb₂(WO₄)₃ | >99 | 0 | 20 | 73 |
| 3 | Y₂(WO₄)₃ | >99 | 4 | 34 | 64 |
| 4 | Bi₂(WO₄)₃ | >99 | 0 | 20 | 71 |
| 5 | ZnMoO₄ | >99 | 0 | 13 | 59 |

As shown in Table 6, the conversion of oleic acid is complete after 24 h at 30 °C. The results also suggest that the epoxide observed at t = 5 h is converted into the HPA or the 1,2-diol. Good yields of HPA from 59 to 76 % are obtained with the bimetallic mixed oxides.

### Example 7: Study of various calcined catalysts

Using a procedure analogous to that described in Example 5, the following calcined bimetallic mixed oxides were evaluated as catalysts: c-ZnWO₄, c-Yb₂(WO₄)₃, c-Y₂(WO₄)₃, c-Al₂(WO₄)₃, c-Bi₂(WO₄)₃ and c-Zr(WO₄)₂. The reaction was performed at 30 °C for 24 h (Table 7) with oleic acid (250 mg, 0.8 mmol, 1.0 equiv.), in the presence of H₂O₂ (60 % (m/V), 0.159 mL, 2.8 mmol, 3.5 equiv.) in acetic acid (0.75 mL).

**Table 7: Results of calcined bimetallic mixed oxides catalytic activity after 24 h at 30 °C.**

| **Entry** | **Catalyst** | **OA conv. (%)** | **Epoxide (%)** | **1,2-diol (%)** | **HPA Ia/b (%)** |
|---|---|---|---|---|---|
| 1 | c-ZnWO₄ | >99 | 0 | 26 | 64 |
| 2 | c-Yb₂(WO₄)₃ | >99 | 0 | 23 | 81 |
| 3 | c-Y₂(WO₄)₃ | >99 | 0 | 24 | 80 |
| 4 | c-Al₂(WO₄)₃ | >99 | 2 | 27 | 61 |
| 5 | c-Bi₂(WO₄)₃ | >99 | 0 | 34 | 67 |
| 6 | c-Zr(WO₄)₂ | 88 | 8 | 30 | 50 |

As shown in Table 7, the calcined bimetallic mixed oxides allow to obtain a complete or almost complete conversion of oleic acid after 24 h at 30 °C. HPA was obtained in good yields from 50 to 81 %, as a major product. The 1,2-diol of oleic acid was also obtained in yields from 23 to 34 %. The epoxide was also observed in trace amounts with c-Al₂(WO₄)₃ and c-Zr(WO₄)₂.

### Example 8: Comparative experiment

A blank reaction (without catalyst) was done using a procedure analogous to that described in Example 5. The reaction was performed at 30 °C for 5 h with oleic acid (250 mg, 0.8 mmol, 1.0 equiv.), in the presence of 3.5 equiv. of H₂O₂ (60 % (m/V), 0.159 mL, 2.8 mmol, 3.5 equiv.) in acetic acid (0.75 mL). The results are presented in Table 8.

**Table 8: Results of the reaction in presence and absence of ZnWO₄ catalyst at 30 °C for 5h.**

| Entry | Catalyst | Epoxide (%) | 1,2-Diol (%) | HPA **Ia/b** (%) | OA Conv. (%) |
|---|---|---|---|---|---|
| 1 | Without catalyst | 9 | 7 | 9 | 34 |
| 2 | ZnWO₄ | 0 | 19 | 70 | >99 |

Table 8 shows that a poor oleic acid (OA) conversion of 34% and a poor yield of 9 % in HPA were obtained in the absence of a catalyst, after 5 h at 30 °C. On the contrary, the use of a bimetallic mixed oxide such as ZnWO₄ as a catalyst allows to obtain a complete OA conversion and a very good yield of 70 % in HPA.

### Example 9: Experiment with H₂O₂ of different concentrations (60 % and 70 %)

Using the procedure described in Example 5, the catalytic activity of h-ZnWO₄ was tested in the presence of H₂O₂ of three different concentrations: 30 %, 60 % and 70 %. The reactions were performed at 30 °C for 24 h (Table 9) with oleic acid (250 mg, 0.8 mmol, 1.0 equiv.), in the presence of 3.5 equiv. of H₂O₂ (30 % (m/V) - 0.317 mL; 60 % (m/V) - 0.159 mL; or 70 % (m/V) - 0.136 mL, 2.8 mmol) in acetic acid (0.75 mL) and 20 mg h-ZnWO₄ (8 wt% with respect to the substrate).

**Table 9: Results of the reaction performed with 30%, 60% and 70% H₂O₂ at 30 °C for 24 h.**

| Entry | H₂O (%) | OA conv. (%) | Epoxide (%) | 1,2-diol (%) | HPA Ia/b (%) |
|---|---|---|---|---|---|
| 1 | 30 | >99 | 0 | 30 | 48 |
| 2 | 60 | >99 | 0 | 29 | 62 |
| 3 | 70 | >99 | 0 | 17 | 61 |

Table 9 shows that the use of a H₂O₂ concentration from 30 % to 70 % allows to reach a complete oleic acid (OA) conversion, and to obtain HPA as a major product in 48-62% yields. The 1,2-diol was also obtained in 17-30% yields.

### Example 10: kinetic studies for h-ZnWO₄, ZnWO₄, Yb₂(WO₄)₃ and c-ZnWO₄

Using a procedure analogous to that described in Example 5, a kinetic study was done using several catalysts: h-ZnWO₄ (Table 10), ZnWO₄ (Table 11), Yb₂(WO₄)₃ (Table 12), c-ZnWO₄ (Table 13), and h-ZnMoO₄ (Table 14). The reaction was performed using 8 wt% of catalyst at 30 °C for 5 h with oleic acid (250 mg, 0.8 mmol, 1.0 equiv.), in the presence of H₂O₂ (60 % (m/V), 0.159 mL, 2.8 mmol, 3.5 equiv.) in acetic acid (0.75 mL). Samples were taken at different reaction times (3 h, 4 h, 5 h, 6 h, 7 h, 15 h and 24 h) and analyzed by ¹H-NMR.

**Table 10: Kinetic study using b-ZnWO₄catalyst.**

| Reaction time (h) | Epoxide (%) | 1,2-Diol (%) | HPA **Ia/b** (%) | OA Conv. (%) |
|---|---|---|---|---|
| 3 | 10 | 5 | 7 | 9 |
| 4 | 11 | 6 | 8 | 16 |
| 5 | 12 | 8 | 13 | 18 |
| 6 | 12 | 12 | 15 | 25 |
| 7 | 10 | 10 | 18 | 35 |
| 15 | 8 | 25 | 44 | 70 |
| 24 | 4 | 26 | 54 | 92 |

**Table 11: Kinetic study using ZnWO₄ catalyst.**

| Reaction time (h) | Epoxide (%) | 1,2-Diol (%) | HPA (%) | OA Conv. (%) |
|---|---|---|---|---|
| 3 | 8 | 16 | 64 | 86 |
| 5 | 0 | 19 | 69 | >99 |
| 7 | 0 | 17 | 67 | >99 |
| 24 | 0 | 16 | 60 | >99 |

**Table 12: Kinetic study using Yb₂(WO₄)₃ catalyst.**

| Reaction time (h) | Epoxide (%) | 1,2-Diol (%) | HPA (%) | OA Conv. (%) |
|---|---|---|---|---|
| 3 | 5 | 26 | 68 | 85 |
| 5 | 0 | 28 | 70 | >99 |
| 7 | 0 | 23 | 60 | >99 |
| 15 | 0 | 24 | 71 | >99 |
| 24 | 0 | 21 | 65 | >99 |

**Table 13: Kinetic study using c-ZnWO₄ catalyst.**

| Reaction time (h) | Epoxide (%) | 1,2-Diol (%) | HPA (%) | OA Conv. (%) |
|---|---|---|---|---|
| 3 | 11 | 3 | 5 | 10 |
| 5 | 14 | 6 | 9 | 20 |
| 7 | 15 | 8 | 13 | 31 |
| 15 | 9 | 19 | 42 | 73 |
| 24 | 6 | 24 | 52 | 93 |

**Table 14: Kinetic study using h-ZnMoO₄ catalyst.**

| Reaction time (h) | Epoxide (%) | 1,2-Diol (%) | HPA (%) | OA Conv. (%) |
|---|---|---|---|---|
| 1 | 10 | 0 | 5 | 9 |
| 3 | 18 | 5 | 22 | 53 |
| 5 | 7 | 9 | 45 | 86 |
| 7 | 4 | 14 | 59 | 96 |
| 24 | 0 | 14 | 56 | >99 |

The kinetics studies show that a complete oleic acid (OA) conversion is reached after 5 h only with ZnWO₄ and Yb₂(WO₄)₃ catalysts, and leads to HPA yields around 60-70% (Tables 11 and 12). A longer reaction time (24 h) is required with h-ZnMoO₄, c-ZnWO₄, and h-ZnWO₄, and HPA yields around 50-60 % are obtained (Tables 10, 13 and 14). Tables 10, 13 and 14 also show that a small amount of epoxide is formed throughout the first hours of the reaction, and is then converted into 1,2-diol or HPA.

### Example 11: Study of the recycling of ZnWO₄

The recycling of the Zn WO₄ catalyst was performed following a procedure analogous to that described in Example 5. The reaction was performed using ZnWO₄ (first catalytic loading: 8 wt%) at 30 °C for 5 h with oleic acid (250 mg, 0.8 mmol, 1.0 equiv.), in the presence of H₂O₂ (60 % (m/V), 0.159 mL, 2.8 mmol, 3.5 equiv.) in acetic acid (0.75 mL). Upon completion of the reaction, a sample was taken and analyzed by ¹HNMR. Afterwards, the reaction mixture was transferred into 50 mL Teflon centrifuge tubes and the catalyst ZnWO₄ was separated by centrifugation at 6000 rpm during 20 min. After the centrifugation, the catalyst was washed with ethanol (pure 96 °). The centrifugation-washing step was repeated 3 times. Next, ZnWO₄ was dried in air atmosphere at 120 °C during 12 h before to be reusing in the next reaction run. (Table 15)

**Table 15: Results of the recycling of the ZnWO₄ catalyst.**

| RUN No. | Epoxide (%) | 1,2-Diol (%) | HPA **Ia/b** (%) | OA Conv. (%) |
|---|---|---|---|---|
| 1 | 0 | 17 | 62 | >99 |
| 2 | 0 | 19 | 63 | >99 |
| 3 | 0 | 18 | 56 | >99 |
| 4 | 0 | 19 | 58 | >99 |
| 5 | 0 | 24 | 53 | >99 |
| 6 | 0 | 23 | 52 | >99 |
| 7 | 0 | 14 | 54 | >99 |
| 8 | 0 | 13 | 57 | >99 |
| 9 | 0 | 23 | 52 | >99 |
| 10 | 0 | 24 | 50 | >99 |
| 11 | 5 | 24 | 32 | 76 |

Table 15 demonstrates that the ZnWO₄ catalyst used in the process of the invention can be efficiently recycled. As a matter of fact, high or complete OA conversions are obtained over at least 11 runs, HPA yields above 60 % are obtained for the first two runs and HPA yields above 50 % are obtained for runs 1 to 10.

### Example 12: Study of the recycling of the catalyst h-ZnWO₄

Using a procedure analogous to that described in Example 5, the following reagent quantities were used: 2.50 g oleic acid (90 % purity) (1.0 equiv.), 1.59 mL H₂O₂ (60 % (m/V) (3.5 equiv.), acetic acid (7.5 mL) and 200 mg h-ZnWO₄ (initial catalyst loading: 8 wt % with respect to the substrate). The mixture was heated at 30 °C under stirring (400 rpm) for 24 h. Upon completion of the reaction, 1,4-dibromobenzene (purity > 90%) (950 mg, 4 mmol) was added to the reaction mixture and then diluted with acetic acid (puriss ≥ 99.8 %) to 50 mL using a volumetric flask. After the qNMR analysis, the mixture was transferred into 50 mL Teflon centrifuge tubes and the catalyst h-ZnWO₄ was separated by centrifugation at 6000 rpm during 20 min. After the centrifugation, the catalyst was washed with ethanol (pure 96°). The centrifugation-washing step was repeated 3 times. Afterwards, b-ZnWO₄ was dried in air atmosphere at 120 °C during 12 h before to be reusing in the next reaction run. (Table 16)

**Table 16: Results of the recycling of the b-ZnWO₄ catalyst.**

| RUN N° | OA conv (%) | 1,2-diol (%) | HPA (%) |
|---|---|---|---|
| 1 | >99 | 29 | 62 |
| 2 | >99 | 29 | 58 |
| 3 | >99 | 29 | 58 |
| 4 | >99 | 27 | 55 |
| 5 | >99 | 28 | 56 |
| 6 | >99 | 29 | 55 |
| 7 | >99 | 31 | 58 |
| 8 | >99 | 31 | 55 |
| 9 | >99 | 27 | 55 |
| 10 | >99 | 29 | 55 |
| 11 | 97 | 27 | 51 |
| 12 | 97 | 26 | 51 |
| 13 | >99 | 27 | 53 |
| 14 | 98 | 25 | 49 |
| 15 | 96 | 30 | 54 |
| 16 | 95 | 29 | 55 |
| 17 | 91 | 13 | 47 |
| 18 | 95 | 30 | 54 |
| 19 | 96 | 25 | 49 |
| 20 | 93 | 26 | 47 |
| 21 | 92 | 24 | 41 |
| 22 | 88 | 24 | 38 |

Table 16 demonstrates that the h-Zn WO₄ catalyst used in the process of the invention can be efficiently recycled. As a matter of fact, high or complete oleic acid (OA) conversions are obtained over at least 22 runs, HPA yields of at least 55 % are obtained for runs 1 to 10 and HPA yields between 40 and 55 % are obtained for runs 11 to 21.

### Example 13: Catalytic activity of h-Zn WO₄ using different substrates

Following the procedure described in Example 5 using h-Zn WO₄ as the catalyst, triolein (> 80 %), olive oil and cyclohexene were used as substrates. The reaction was performed using b-ZnWO₄(8 wt%) at 30 °C for 24 h with the substrate (0.8 mmol, 1.0 equiv. of C=C double bonds), in the presence of H₂O₂ (60 % (m/V), 0.159 mL, 2.8 mmol, 3.5 equiv. relative to C=C double bonds) in acetic acid (0.75 mL). In the case of triolein and olive oil, a triple quantity of H₂O₂ (10.5 equiv. relative to the substrate) was used since it contains triple amount of double bounds compared with oleic acid. In the case of cyclohexene, the following quantities were used: 66 mg of cyclohexene (0.8 mmol, 1 equiv.), H₂O₂ (60 % (m/V), 0.159 mL, 2.8 mmol, 3.5 equiv.), acetic acid (0.75 mL), and 5.2 mg h-ZnWO₄ (8 wt%). The results are presented in Table 17.

**Table 17: Results of h-Zn WO₄ catalytic activity using different substrates.**

| Entry | Substrate | Epoxide (%) | 1,2-Diol (%) | HPA (%) | Substrate Conv. (%) |
|---|---|---|---|---|---|
| 1 | Oleic acid | 0 | 30 | 62 | >99 |
| 2 | Triolein | 6 | 16 | 46 | 69 |
| 3 | Olive oil | 6 | 21 | 65 | 93 |
| 4 | Cyclohexene | 0 | 26 | 30 | >99 |

As shown in Table 17, the process of the invention can be applied to various olefin substrates. High or complete oleic acid (OA) conversions can be reached and HPAs are obtained as a major product in 30 to 65 % yields.

## Claims

1. A process for preparing hydroperoxy alcohols with the following formula (Ia) and/or (Ib):
R¹-CR⁴(OOH)-CR³(OH)-R² (Ia)
R²-CR³(OOH)-CR⁴(OH)-R¹ (Ib)
wherein R¹ and R² are each independently an optionally substituted alkyl group with 1 to 20 carbon atoms or a -L-A group wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a-(CH₂)-CH(OCOR⁵)-CHOCOR⁶ group, with R⁵ and R⁶ being independently a C8-C22 linear or branched alkyl group which is optionally substituted by at least one hydroperoxy or hydroxyl substituent,
or R¹ and R² form together a carbocycle consisting of a 6 to 12 membered ring which is optionally substituted.
R³and R⁴ are each independently a hydrogen atom, an optionally substituted alkyl group with 1 to 20 carbon atoms or a group -L-A wherein L is a bond or a linear or branched alkylene chain with 1 to 12 carbon atoms, which is optionally substituted, and A is a hydrogen atom or a -COXR group wherein X is an oxygen atom or a -NR' group with R and R' being independently a group selected from a hydrogen atom, a C1-C8 alkyl group, or a -(CH₂)-CH(OCOR⁵)-CHOCOR⁶ group, with R⁵ and R⁶ being independently a C8-C22 linear or branched alkyl group which is optionally substituted by at least one hydroperoxy or hydroxyl substituent,
comprising reacting at least one functionalized or unfunctionalized, linear or cyclic, olefin which comprises at least one carbon - carbon double bond with aqueous hydrogen peroxide in a solvent selected from water-soluble carboxylic acids, in the presence of a heterogenous catalyst which is a metallic mixed oxide having formula (II):
MₓM'_{y}O_{z} (II)
wherein:
M is a metal;
M' is W or Mo,
x is a number from 1 to 10, preferably from 1 to 5, more preferably from 1 to 2;
y is a number from 1 to 10, preferably from 1 to 5, more preferably from 1 to 3;
z is a number from 1 to 40, preferably from 2 to 20, more preferably from 3 to 15.

2. The process according to claim 1, **characterized in that** the metal M is such that its cation forms a Lewis acid and is preferably selected from the group consisting of alkaline earth metals, transition metals, post-transition metals, lanthanides and rare earth elements, more preferably, M is selected from Zn, Yb, Y, Bi, Al, Zr, still more preferably it is selected from Zn, Yb, Y, Bi, and still better M is Zn.

3. The process according to claim 1 or 2, **characterized in that** the process is conducted using 0.1-20 wt%, preferably 1-20 wt%, more preferably 5-10 wt%, even more preferably 7-9 wt% of the catalyst, relative to the weight of the olefin.

4. The process according to any one of claims 1 to 3, **characterized in that** the functionalized olefin is selected from the group consisting of mono- or polyunsaturated aliphatic carboxylic acids and their esters, such as lauroleic acid, myristoleic acid, palmitoleic acid, sapienic acid, petroselaidic acid, oleic acid, elaidic acid, petroselinic acid, vaccenic acid, gadoleic acid, cetoleic acid, erucic acid, selacholeic or nervonic acid, linoleic acid, linolenic acid, rumenic acid, stearidonic acid, eleostearic acid, catalpic acid, arachidonic acid, and their mixtures, preferably the functionalized olefin is selected from oleic acid, palmitoleic acid, erucic acid, linoleic acid, linolenic acid, their esters and mixtures thereof, more preferably the functionalized olefin is oleic acid or one of its esters, more particularly methyl oleate.

5. The process according to claim 4, **characterized in that** the ester of the acid consists of one or more triglycerides derived from a vegetable oil.

6. The process according to any one of claims 1 to 5, **characterized in that** hydrogen peroxide is used as an aqueous solution having a concentration of at least 30 %, or at least 45 % and at most 60 % or even 70 %, for example 30 %, 45 %, 60 % or 70 % (m/v).

7. The process according to any one of claims 1 to 6, **characterized in that** the quantity of hydrogen peroxide at 60 % (m/V) used in the process ranges from 2 to 10 equivalents, preferably from 3.5 to 4 equivalents and more preferably is about 3.5 equivalents.

8. The process according to any one of claims 1 to 7, **characterized in that** the solvent is selected from acetic acid and formic acid and is preferably acetic acid.

9. The process according to any one of claims 1 to 8, **characterized in that** the quantity of solvent used ranges from 0.1 to 100 mL/mmol of olefin, preferably from 0.5 to 10 mL/mmol of olefin and more preferably from 0.9 to 1 mL/mmol of olefin.

10. The process according to any one of claims 1 to 9, **characterized in that** the process is performed at a temperature of 0 - 120 °C, preferably 0 - 90 °C, more preferably of 20 - 60 °C and even more preferably of from 25 to 35 °C.

11. The process according to any one of claims 1 to 10, **characterized in that** it further comprises the steps of:
- separating the catalyst from the reaction medium, washing it with an alcohol, wherein the centrifugation and washing steps may be repeated 1-10 times, and optionally drying the catalyst, and
- recycling the catalyst in a process as defined in claim 1.

12. Use of the catalyst as defined in any one of claims 1 to 3 in the synthesis of hydroperoxy alcohols.

## Patentansprüche

1. Verfahren zur Herstellung von Hydroperoxyalkoholen mit der folgenden Formel (Ia) und/oder (Ib):
R¹-CR⁴ (OOH) -CR³ (OH) -R² (Ia)
R²-CR³ (00H) -CR⁴ (OH) -R¹ (Ib)
wobei:
R¹ und R² jeweils unabhängig für eine gegebenenfalls substituierte Alkylgruppe mit 1 bis 20 Kohlenstoffatomen oder eine Gruppe -L-A stehen, wobei L für eine Bindung oder eine lineare oder verzweigte Alkylenkette mit 1 bis 12 Kohlenstoffatomen, die gegebenenfalls substituiert ist, steht und A für ein Wasserstoffatom oder eine Gruppe -COXR steht, in der X für ein Sauerstoffatom oder eine Gruppe -NR' steht, wobei R und R' jeweils unabhängig für eine Gruppe stehen, die aus einem Wasserstoffatom, einer C₁-C₈-Alkylgruppe oder einer Gruppe - (CH₂) -CH (OCOR⁵) - CHOCOR⁶ ausgewählt ist, wobei R⁵ und R⁶ jeweils unabhängig für eine lineare oder verzweigte C₈-C₂₂-Alkylgruppe, die gegebenenfalls durch mindestens einen Hydroperoxy- oder Hydroxysubstituenten substituiert ist, stehen,
oder R¹ und R² zusammen einen Carbocyclus bilden, der aus einem 6- bis 12-gliedrigen Ring besteht, der gegebenenfalls substituiert ist,
R³ und R⁴ jeweils unabhängig für ein Wasserstoffatom oder eine gegebenenfalls substituierte Alkylgruppe mit 1 bis 20 Kohlenstoffatomen oder eine Gruppe -L-A stehen, wobei L für eine Bindung oder eine lineare oder verzweigte Alkylenkette mit 1 bis 12 Kohlenstoffatomen, die gegebenenfalls substituiert ist, steht und A für ein Wasserstoffatom oder eine Gruppe -COXR steht, in der X für ein Sauerstoffatom oder eine Gruppe -NR' steht, wobei R und R' jeweils unabhängig für eine Gruppe stehen, die aus einem Wasserstoffatom, einer C₁-C₈-Alkylgruppe oder einer Gruppe -(CH₂)-CH(OCOR⁵)-CHOCOR⁶ ausgewählt ist, wobei R⁵ und R⁶ jeweils unabhängig für eine lineare oder verzweigte C₈-C₂₂-Alkylgruppe, die gegebenenfalls durch mindestens einen Hydroperoxy- oder Hydroxysubstituenten substituiert ist, stehen,
umfassend das Umsetzen mindestens eines funktionalisierten oder unfunktionalisierten, linearen oder cyclischen Olefins mit mindestens einer Kohlenstoff-Kohlenstoff-Doppelbindung mit wässrigem Wasserstoffperoxid in einem aus wasserlöslichen Carbonsäuren ausgewählten Lösungsmittel in Gegenwart eines heterogenen Katalysators, bei dem es sich um ein Metallmischoxid mit der Formel (II) handelt:
MₓM'_{y}O_{z} (II),
wobei:
M für ein Metall steht;
M' für W oder Mo steht;
x für eine Zahl von 1 bis 10, vorzugsweise von 1 bis 5, weiter bevorzugt von 1 bis 2, steht;
y für eine Zahl von 1 bis 10, vorzugsweise von 1 bis 5, weiter bevorzugt von 1 bis 3, steht;
z für eine Zahl von 1 bis 40, vorzugsweise von 2 bis 20, weiter bevorzugt von 3 bis 15, steht.

2. Verfahren nach Anspruch 1, **dadurch gekennzeichnet, dass** das Metall M so beschaffen ist, dass sein Kation eine Lewis-Säure bildet, und vorzugsweise aus der Gruppe bestehend aus Erdalkalimetallen, Übergangsmetallen, Nachübergangsmetallen Lanthaniden und Seltenerdelementen ausgewählt ist, M weiter bevorzugt aus Zn, Yb, Y, Bi, Al, Zr ausgewählt ist und noch weiter bevorzugt aus Zn, Yb, Y, Bi, ausgewählt ist und M noch besser für Zn steht.

3. Verfahren nach Anspruch 1 oder 2, **dadurch gekennzeichnet, dass** das Verfahren unter Verwendung von 0,1-20 Gew.-%, vorzugsweise 1-20 Gew.-%, weiter bevorzugt 5-10 Gew.-%, noch weiter bevorzugt 7-9 Gew.-%, des Katalysators, bezogen auf das Gewicht des Olefins, durchgeführt wird.

4. Verfahren nach einem der Ansprüche 1 bis 3, **dadurch gekennzeichnet, dass** das funktionalisierte Olefin aus der Gruppe bestehend aus ein- oder mehrfach ungesättigten aliphatischen Carbonsäuren und deren Estern, wie Lauroleinsäure, Myristoleinsäure, Palmitoleinsäure, Sapiensäure, Petroselaidinsäure, Ölsäure, Elaidinsäure, Petroselinsäure, Vaccensäure, Gadoleinsäure, Cetoleinsäure, Erucasäure, Selacholeinsäure oder Nervonsäure, Linolsäure, Linolensäure, Rumensäure, Stearidonsäure, Eleostearinsäure, Catalpinsäure, Arachidonsäure und Mischungen davon ausgewählt wird, das funktionalisierte Olefin vorzugsweise aus Ölsäure, Palmitoleinsäure, Erucasäure, Linolensäure, Linolensäure, Estern davon und Mischungen davon ausgewählt ist und das funktionalisierte Olefin weiter bevorzugt Ölsäure oder einer ihrer Ester, spezieller Methyloleat, ist.

5. Verfahren nach Anspruch 4, **dadurch gekennzeichnet, dass** der Ester der Säure aus einem oder mehreren von einem Pflanzenöl abgeleiteten Triglyceriden besteht.

6. Verfahren nach einem der Ansprüche 1 bis 5, **dadurch gekennzeichnet, dass** das Wasserstoffperoxid in Form einer wässrigen Lösung mit einer Konzentration von mindestens 30 % oder mindestens 45 % und höchstens 60 % oder sogar 70 %, beispielsweise 30 %, 45 %, 60 % oder 70 % (m/v), verwendet wird.

7. Verfahren nach einem der Ansprüche 1 bis 6, **dadurch gekennzeichnet, dass** die bei dem Verfahren verwendete Menge an Wasserstoffperoxid in einer Konzentration von 60 % (m/V) im Bereich von 2 bis 10 Äquivalenten, vorzugsweise von 3,5 bis 4 Äquivalenten, liegt und weiter bevorzugt etwa 3,5 Äquivalente beträgt.

8. Verfahren nach einem der Ansprüche 1 bis 7, **dadurch gekennzeichnet, dass** das Lösungsmittel aus Essigsäure und Ameisensäure ausgewählt wird und vorzugsweise Essigsäure ist.

9. Verfahren nach einem der Ansprüche 1 bis 8, **dadurch gekennzeichnet, dass** die verwendete Lösungsmittelmenge im Bereich von 0,1 bis 100 ml/mmol Olefin, vorzugsweise von 0,5 bis 10 ml/mmol Olefin und weiter bevorzugt 0,9 bis 1 ml/mmol Olefin liegt.

10. Verfahren nach einem der Ansprüche 1 bis 9, **dadurch gekennzeichnet, dass** das Verfahren bei einer Temperatur von 0-120 °C, vorzugsweise 0-90 °C, weiter bevorzugt 20-60 °C und noch weiter bevorzugt 25 bis 35 °C durchgeführt wird.

11. Verfahren nach einem der Ansprüche 1 bis 10, **dadurch gekennzeichnet, dass** es ferner folgende Schritte umfasst:
- Abtrennen des Katalysators vom Reaktionsmedium und Waschen des Katalysators mit einem Alkohol, wobei die Zentrifugations- und Waschschritte 1-10-mal wiederholt werden können, und gegebenenfalls Trocknen des Katalysators und
- Zurückführen des Katalysators in ein Verfahren gemäß Anspruch 1.

12. Verwendung des Katalysators gemäß einem der Ansprüche 1 bis 3 bei der Synthese von Hydroperoxyalkoholen.

## Revendications

1. Procédé de préparation d'hydroperoxyalcools de formule (Ia) et/ou (Ib):
R¹-CR⁴(OOH)-CR³(OH)-R² (la)
R²-CR³(OOH)-CR⁴(OH)-R¹ (Ib)
où R¹ et R² sont chacun indépendamment un groupe alkyle de 1 à 20 atomes de carbone éventuellement substitué ou un groupe -L-A dans lequel L est une liaison ou une chaîne alkylène linéaire ou ramifiée ayant de 1 à 12 atomes de carbone, qui est éventuellement substituée, et A est un atome d'hydrogène ou un groupe -COXR où X est un atome d'oxygène ou un groupe -NR' dans lequel R et R' désignent indépendamment un groupe choisi parmi un atome d'hydrogène, un groupe alkyle en C1-C8, ou un groupe -(CH₂)-CH(OCOR⁵)-CHOCOR⁶ où R⁵ et R⁶ sont indépendamment un groupe alkyle en C8-22 linéaire ou ramifié, qui est éventuellement substitué par au moins un substituant hydroperoxy ou hydroxyle,
ou bien R¹ et R² forment ensemble un carbocycle consistant en un cycle ayant de 6 à 12 chaînons qui est éventuellement substitué
R³ et R⁴ sont chacun indépendamment un atome d'hydrogène, un groupe alkyle ayant de 1 à 20 atomes de carbone, éventuellement substitué, ou un groupe -L-A dans lequel L est une liaison ou une chaîne alkylène linéaire ou ramifiée ayant de 1 à 12 atomes de carbone, qui est éventuellement substituée, et A est un atome d'hydrogène ou un groupe -COXR où X est un atome d'oxygène ou un groupe -NR' dans lequel R et R' désignent indépendamment un groupe choisi parmi un atome d'hydrogène, un groupe alkyle en C1-C8, ou un groupe -(CH₂)-CH(OCOR⁵)-CHOCOR⁶ où R⁵ et R⁶ sont indépendamment un groupe alkyle en C8-22 linéaire ou ramifié qui est éventuellement substitué par au moins un substituant hydroperoxy ou hydroxyle,
comprenant la réaction d'au moins une oléfine fonctionnalisée ou non fonctionnalisée, linéaire ou cyclique, comprenant au moins une double liaison carbone-carbone, avec du peroxyde d'hydrogène aqueux, dans un solvant choisi parmi les acides carboxyliques hydrosolubles, en présence d'un catalyseur hétérogène qui est un oxyde mixte métallique de formule (II):
MₓM'_{y}O_{z} (II)
dans laquelle:
M est un métal;
M' est W ou Mo,
x est un nombre de 1 à 10, de préférence de 1 à 5, plus préférentiellement de 1 à 2 ;
y est un nombre de 1 à 10, de préférence de 1 à 5, plus préférentiellement de 1 à 3 ;
z est un nombre de 1 à 40, de préférence de 2 à 20, plus préférentiellement de 3 à 15.

2. Procédé selon la revendication 1, **caractérisé en ce que** le métal M est tel que son cation forme un acide de Lewis et est de préférence choisi dans le groupe constitué des métaux alcalino-terreux, des métaux de transition, des métaux de post-transition, des lanthanides et des terres rares, plus préférentiellement M est choisi parmi Zn, Yb, Y, Bi, Al, Zr, mieux, il est choisi parmi Zn, Yb, Y, Bi, et encore mieux, M est Zn.

3. Procédé selon la revendication 1 ou 2, **caractérisé en ce que** le procédé est conduit en utilisant 0,1-20% en poids, de préférence 1-20% en poids, plus préférentiellement 5-10% en poids, mieux, 7-9% en poids de catalyseur, par rapport au poids de l'oléfine.

4. Procédé selon l'une quelconque des revendications 1 à 3, **caractérisé en ce que** l'oléfine fonctionnalisée est choisie dans le groupe constitué des acides carboxyliques aliphatiques mono- ou polyinsaturés et leurs esters, tels que l'acide lauroléique, l'acide myristoléique, l'acide palmitoléique, l'acide sapiénique, l'acide pétrosélaïdique, l'acide oléique, l'acide élaïdique, l'acide pétrosélinique, l'acide vaccénique, l'acide gadoléique, l'acide cétoléique, l'acide érucique, l'acide sélacholéique ou l'acide nervonique, l'acide linoléique, l'acide linolénique, l'acide ruménique, l'acide stéaridonique, l'acide éléostéarique, l'acide catalpique, l'acide arachidonique, et leurs mélanges, de préférence l'oléfine fonctionnalisée est choisie parmi l'acide oléique, l'acide palmitoléique, l'acide érucique, l'acide linoléique, l'acide linolénique, leurs esters et leurs mélanges, plus préférentiellement l'oléfine fonctionnalisée est l'acide oléique ou l'un de ses esters, mieux, l'oléate de méthyle.

5. Procédé selon la revendication 4, **caractérisé en ce que** l'ester de l'acide consiste en un ou plusieurs triglycérides dérivé(s) d'une huile végétale.

6. Procédé selon l'une quelconque des revendications 1 à 5, **caractérisé en ce que** le peroxyde d'hydrogène est utilisé sous forme d'une solution aqueuse ayant une concentration d'au moins 30% ou d'au moins 45% et d'au plus 60% ou même 70%, par exemple de 30%, 45%, 60% ou 70% (m/v).

7. Procédé selon l'une quelconque des revendications 1 à 6, **caractérisé en ce que** la quantité de peroxyde d'hydrogène à 60% (m/V) utosiée dans le procédé va de 2 à 10 équivalents, de préférence de 3,5 à 4 équivalents et, mieux, est d'environ 3,5 équivalents.

8. Procédé selon l'une quelconque des revendications 1 à 7, **caractérisé en ce que** le solvant est choisi parmi l'acide acétique et l'acide formique et est de préférence l'acide acétique.

9. Procédé selon l'une quelconque des revendications 1 à 8, **caractérisé en ce que** la quantité de solvant utilisée va de 0,1 à 100 mL/mmol d'oléfine, de préférence de 0,5 à 10 mL/mmol d'oléfine et, mieux, de 0,9 à 1 mL/mmol d'oléfine.

10. Procédé selon l'une quelconque des revendications 1 à 9, **caractérisé en ce que** le procédé est effectué à une température de 0 - 120 °C, de préférence de 0 - 90 °C, plus préférentiellement de 20 - 60 °C et, mieux, de 25 à 35 °C.

11. Procédé selon l'une quelconque des revendications 1 à 10, **caractérisé en ce qu'**il comprend en outre les étapes consistant à :
- séparer le catalyseur du milieu réactionnel, le laver avec un alcool, les étapes de centrifugation et de lavage pouvant être répétées de 1 à 10 fois, et éventuellement sécher le catalyseur, et
- recycler le catalyseur dans un procédé tel que défini dans la revendication 1.

12. Utilisation du catalyseur tel que défini dans l'une quelconque des revendications 1 à 3 dans la synthèse d'hydroperoxyalcools.
